(19) Europäisches Patentamt
European Patent Office
Office européen des brevets

(11) EP 4 302 766 A1

(12) EUROPEAN PATENT APPLICATION
published in accordance with Art. 153(4) EPC

(43) Date of publication:
10.01.2024 Bulletin 2024/02

(21) Application number: 21928643.2

(22) Date of filing: 02.04.2021

(51) International Patent Classification (IPC):
*A61K 31/7032* (2006.01) *A61P 37/02* (2006.01)
*A61P 37/00* (2006.01) *C07H 15/10* (2006.01)
*C07H 1/08* (2006.01)

(52) Cooperative Patent Classification (CPC):
**A61K 31/7032; A61P 37/00; A61P 37/02; C07H 1/08; C07H 15/10**

(86) International application number:
**PCT/CN2021/085308**

(87) International publication number:
**WO 2022/183562 (09.09.2022 Gazette 2022/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 03.03.2021 CN 202110235320

(71) Applicant: **Dongguan Hec Cordyceps R&D Co., Ltd.**
**Dongguan, Guangdong 523871 (CN)**

(72) Inventors:
- **GAO, Hao**
  **Dongguan, Guangdong 523871 (CN)**
- **QIAN, Zhengming**
  **Dongguan, Guangdong 523871 (CN)**
- **WANG, Chuanxi**
  **Dongguan, Guangdong 523871 (CN)**
- **JIANG, Shutai**
  **Dongguan, Guangdong 523871 (CN)**
- **YAO, Xinsheng**
  **Dongguan, Guangdong 523871 (CN)**
- **LI, Wenjia**
  **Dongguan, Guangdong 523871 (CN)**
- **HUANG, Qi**
  **Dongguan, Guangdong 523871 (CN)**

(74) Representative: **Hoffmann Eitle**
**Patent- und Rechtsanwälte PartmbB**
**Arabellastraße 30**
**81925 München (DE)**

# (54) GLUCOCEREBROSIDE COMPOUND, PHARMACEUTICAL COMPOSITION THEREOF AND USE THEREOF

(57) A glucocerebroside compound, a pharmaceutical composition thereof, and the use of the glucocerebroside compound and the pharmaceutical composition thereof in the preparation of drugs for preventing or treating immune-related diseases.

EP 4 302 766 A1

## Description

### FIELD OF THE INVENTION

The invention belongs to the field of medicine, and specifically relates to a glucocerebroside compound, a pharmaceutical composition thereof and use thereof. Further, the glucocerebroside compound and the pharmaceutical composition thereof can be used as a drug for preventing or treating immune-related diseases.

### BACKGROUND ART

Cerebroside or glycoceramide belongs to glycosphingolipids, mainly found in animals. It is also distributed in plants and microorganisms. Cerebroside is composed of three parts: glycosyl, fatty acid chain and sphingosine chain. Although these compounds are all composed of three parts: glycosyl, fatty acid chain and sphingosine chain, they have structural diversity due to different types of sugars, different lengths and branching degrees of glycosidic bonds, fatty acid chains and sphingosine chains, different number and positions of hydroxyl groups and double bonds, etc.(J. Nat. Prod. 2017, 80, 6, 1734-1741) Compounds with different structures often have different types or degrees of pharmacological activity, so cerebroside compounds have been reported to have different degrees of antitumor, antiviral, antimicrobial, and neuroprotective activities (Xu Jie, Studies on the Purification, Analysis and Bioactivities of Cerebrosides from Sea Cucumbers, PhD thesis of Ocean University of China, 2011). Most of the glycosyls in cerebrosides are galactose and glucose, so they are divided into galactosylcerebrosides and glucocerebrosides according to the type of glycosyls. The earliest discovery was galactocerebroside, and its structure and activity were reported more, while the glucocerebroside discovered later was relatively less reported.

Cordyceps sinensis is a dry complex of the stromata and larval corpses of the ergot fungus *Cordyceps sinensis* (BerK.) Sacc. parasitizing on the larvae of bat moths. It has various pharmacological effects such as regulating immunity, anti-tumor, anti-oxidation, and anti-aging. Literature reports that the chemical constituents of Cordyceps sinensis mainly include polysaccharides, proteins, sugar alcohols, nucleosides, amino acids, sterols and organic acids. Extracting new active ingredients from Cordyceps sinensis can provide scientific basis for the comprehensive quality evaluation of Cordyceps sinensis and the development and application of related products.

### SUMMARY

The applicant of the present application conducted an in-depth analysis of the ethyl acetate part of Cordyceps sinensis, found that it contained cerebroside components, and they were separated and purified to obtain 4 new cerebroside compound monomers. The immunomodulatory activity of these cerebroside monomers was studied, and it was found that they had excellent immunosuppressive activity.

The purpose of the present invention is to provide a new class of cerebroside compound with immunosuppressive activity and the pharmaceutical composition thereof. The compound and the pharmaceutical composition thereof are used to prevent or treat immune-related diseases.

In one aspect, the present invention provides a compound having the structure described in general formula (I), (II), (III) or (IV), or a solvate, a metabolite, a pharmaceutically acceptable salt or a prodrug thereof,

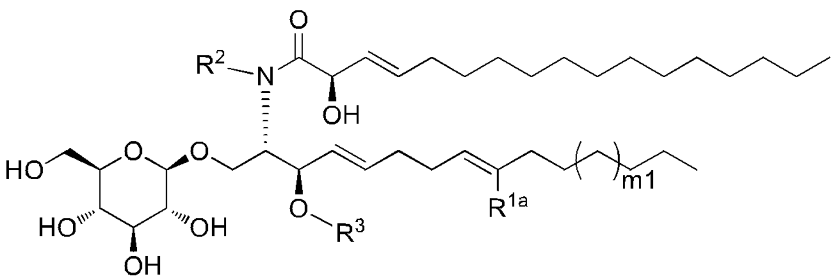

(I),

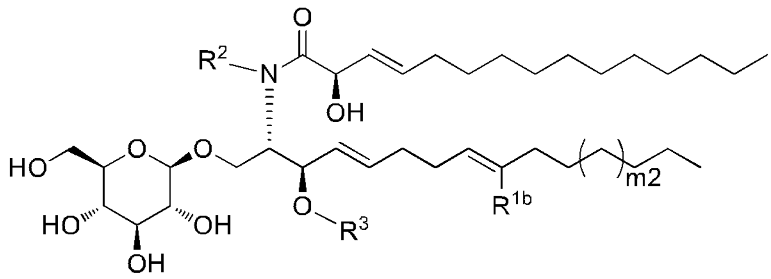

(II),

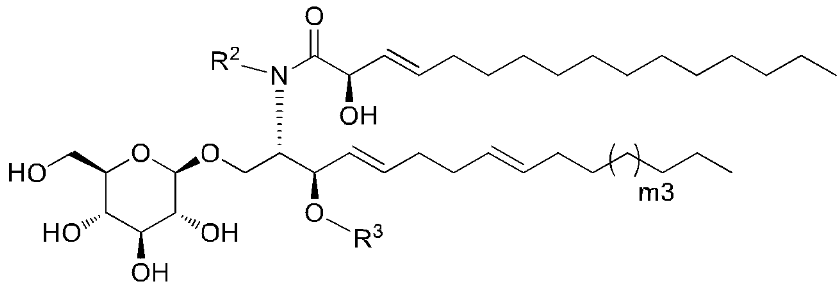

(III),

or

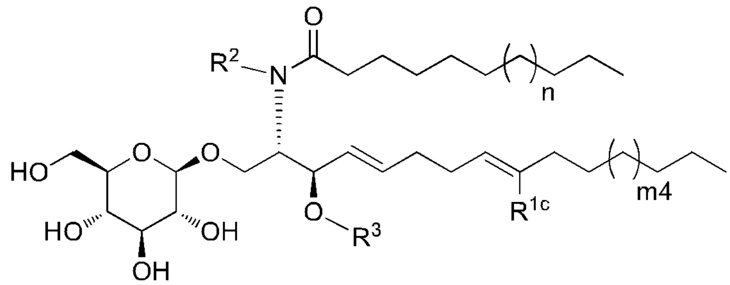

(IV),

wherein,

m1 is 3, 4 or 6;
each of m2, m3 and m4 is independently 3, 4, 5 or 6;
n is 5, 6 or 7;
each of $R^{1a}$ and $R^{1b}$ is independently H, $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl;
$R^{1c}$ is $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl;
each $R^2$ is independently H, methyl, ethyl, n-propyl, isopropyl, n-butyl, isobutyl or *tert-butyl;*
each $R^3$ is independently H, $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl.

In some embodiments, each of $R^{1a}$ and $R^{1b}$ is independently H, methyl, ethyl, *n*-propyl, isopropyl, *n*-butyl, isobutyl, *tert*-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

In some embodiments, $R^{1c}$ is methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and

$-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

In some embodiments, each $R^3$ is independently H, methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, *tert*-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

In some embodiments, the compound of the present invention has any one of the following structures, or a solvate, a metabolite, a pharmaceutically acceptable salt or a prodrug thereof,

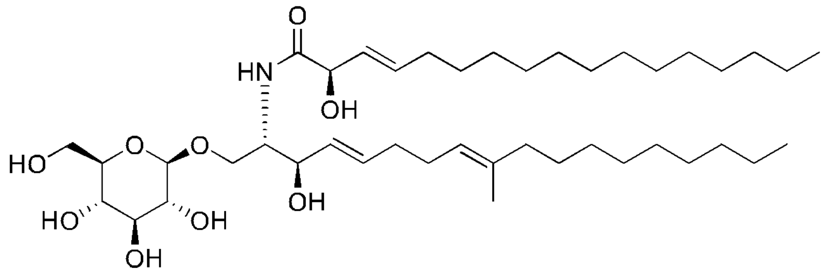

**1**

,

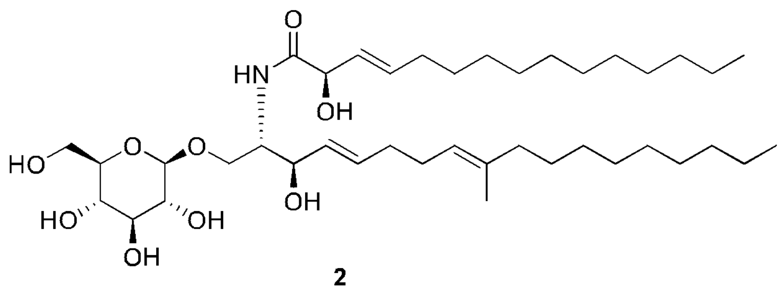

**2**

,

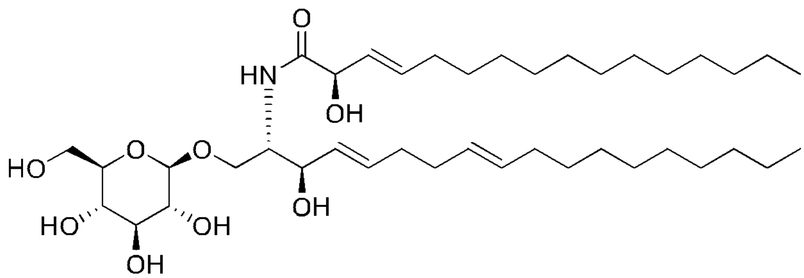

**3**

,

or

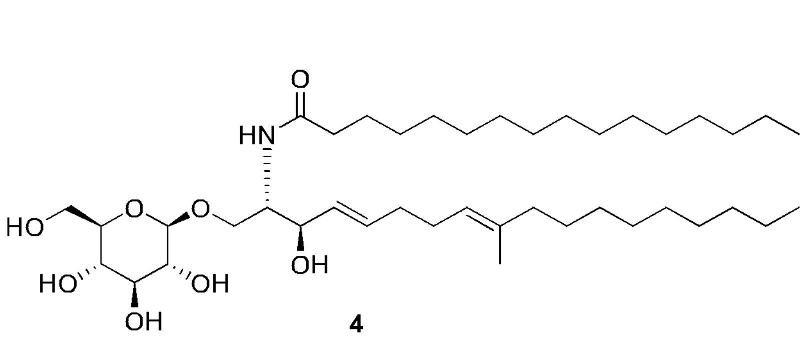

**4**

.

In another aspect, the present invention provides a pharmaceutical composition, which comprises the compound described in the present invention, and a pharmaceutically acceptable adjuvant.

In some embodiments, the pharmaceutical composition of the present invention further comprises other additional therapeutic agent selected from a chemotherapeutic agent or anti-proliferative agent, an anti-inflammatory drug, an immunomodulatory or immunosuppressive agent, a neurotrophic factor, an agent for treating cardiovascular disease, an agent for treating diabetes, and an agent for treating immunodeficiency disorders.

In another aspect, the present invention provides the use of the compound of the present invention and the pharmaceutical composition of the present invention in the manufacture of a medicament for preventing or treating immune-related diseases.

In some embodiments, the immune-related disease is organ transplantation anti-rejection or autoimmune disease, wherein the autoimmune disease is lupus, multiple sclerosis, amyotrophic lateral sclerosis, rheumatoid arthritis, psoriasis, complications from organ transplantation, xeno transplantation, diabetes, asthma, atopic dermatitis, autoimmune thyroid disorders, ulcerative colitis, Crohn's disease, Alzheimer's disease, leukemia or lymphoma.

In another aspect, the present invention provides the use of the compound of the present invention and the pharmaceutical composition of the present invention in the manufacture of a medicament as an immunosuppressant.

In another aspect, the present invention also provides the compound of the present invention or the pharmaceutical composition of the present invention for use in preventing or treating immune-related diseases.

In some embodiments, the immune-related disease is organ transplantation anti-rejection or autoimmune disease, wherein the autoimmune disease is lupus, multiple sclerosis, amyotrophic lateral sclerosis, rheumatoid arthritis, psoriasis, complications from organ transplantation, xeno transplantation, diabetes, asthma, atopic dermatitis, autoimmune thyroid disorders, ulcerative colitis, Crohn's disease, Alzheimer's disease, leukemia or lymphoma.

In another aspect, the present invention also provides the compound of the present invention and the pharmaceutical composition of the present invention for use as an immunosuppressant.

In another aspect, the present invention also provides a method of preventing or treating immune-related diseases comprising administering a therapeutically effective amount of the compound of the present invention or the pharmaceutical composition thereof to a subject.

In some embodiments, the immune-related disease is organ transplantation anti-rejection or autoimmune disease, wherein the autoimmune disease is lupus, multiple sclerosis, amyotrophic lateral sclerosis, rheumatoid arthritis, psoriasis, complications from organ transplantation, xeno transplantation, diabetes, asthma, atopic dermatitis, autoimmune thyroid disorders, ulcerative colitis, Crohn's disease, Alzheimer's disease, leukemia or lymphoma.

In some embodiments, the adjuvant described in the present invention includes, but is not limited to, carrier, excipient, diluent, vehicle, or combinations thereof. In some embodiments, the pharmaceutical composition can be in the form of a liquid, solid, semi-solid, gel or spray.

In certain embodiments, the salt is a pharmaceutically acceptable salt. The term "pharmaceutically acceptable" means that the substance or composition must be chemically and/or toxicologically compatible with the other ingredients comprising the preparation and/or with the mammal being treated therewith.

The compounds of the invention, including their salts, may also be obtained in the form of their hydrates, or include other solvents used for their crystallization. The compounds of the present invention may inherently or by design form solvates with pharmaceutically acceptable solvents (including water); therefore, the invention also include solvated and unsolvated forms.

In other aspect, the present invention relates to a method of preparing, separating or purifying the compound of Formula (I), (II), (III) or (IV).

Compared with prior art, positive effect of the present invention is:

The present application discovers new cerebroside compounds in Cordyceps sinensis for the first time, and these compounds have excellent immunosuppressive activity.

The foregoing merely summarizes certain aspects disclosed herein and is not intended to be limiting in nature. These aspects and other aspects and embodiments are described more fully below.

## DETAILED DESCRIPTION OF THE INVENTION

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application (including but not limited to defined terms, term usage, described techniques, or the like), this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As described herein, compounds disclosed herein may be independently and optionally substituted with one or more substituents, such as are illustrated generally above, or as exemplified by particular classes, subclasses, and species of the invention. It will be appreciated that the phrase "independently and optionally substituted" is used interchangeably with the phrase "substituted or unsubstituted". In general, the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position.

In addition, it should be noted that, unless otherwise clearly stated, the descriptions used in the present invention "each...is independently" can be interchanged with "each of...and...is independently". It should be understood in a broad sense. It can mean that the specific options expressed by the same symbol are independent of each other in different groups; or the specific options expressed by the same symbol are independent of each other in same groups.

At various places in the present specification, substituents of compounds disclosed herein are disclosed in groups or in ranges. It is specifically intended that the invention include each and every individual subcombination of the members of such groups and ranges. For example, the term "$C_{1-6}$ alkyl" is specifically intended to individually disclose methyl, ethyl, $C_3$ alkyl, $C_4$ alkyl, $C_5$ alkyl, and $C_6$ alkyl.

The term "alkyl" or "alkyl group" refers to a saturated linear or branched-chain monovalent hydrocarbon group, wherein the alkyl group is optionally substituted with one or more substituents described herein. In one embodiment of the invention, the alkyl group contains 1-6 carbon atoms. In another embodiment, the alkyl group contains 1-4 carbon atoms. In yet another embodiment, the alkyl group contains 1-3 carbon atoms. Examples of alkyl groups include, but are not limited to, methyl (Me, $-CH_3$), ethyl (Et, $-CH_2CH_3$), n-propyl (n-Pr, $-CH_2CH_2CH_3$), isopropyl (i-Pr , $-CH(CH_3)_2$), n-butyl (n-Bu, $-CH_2CH_2CH_2CH_3$), isobutyl (i-Bu, $-CH_2CH(CH_3)_2$), sec-butyl (s-Bu, $-CH(CH_3)CH_2CH_3$), tert-butyl (t-Bu, $-C(CH_3)_3$), n-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH (CH_3)_2$), 3-methyl-1-butyl ($-CH_2CH_2CH(CH_3)_2$), 2-methyl-1-butyl ($-CH_2CH(CH_3)CH_2CH_3$), n-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$), *etc.*

The term "alkenyl" refers to a linear or branched chain monovalent hydrocarbon radical of 2 to 12 carbon atoms, or 2 to 8 carbon atoms, or 2 to 6 carbon atoms, or 2 to 4 carbon atoms, with at least one site of C-C is a $sp^2$ double bond, wherein the alkenyl radical may be independently substituted with one or more substituents described herein, including radicals having "cis" and "trans" orientations, or "Z" and "E" orientations. In an embodiment of the present invention, the alkenyl group contains 2-6 carbon atoms. Specific examples thereof include, but are not limited to, vinyl ($-CH=CH_2$), allyl ($-CH_2CH=CH_2$), $CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$, *etc.*

The term "cycloalkyl" refers to a saturated monovalent carbocyclic ring system. In an embodiment of the present invention, the cycloalkyl group contains 3-6 carbon ring atoms, specific examples of which include cyclopropyl, cyclopentyl and cyclohexyl.

The term "solvate" refers to an association or complex of one or more solvent molecules and a compound disclosed herein. Some non-limiting examples of the solvent that form solvates include water, isopropanol, ethanol, methanol, dimethylsulfoxide (DMSO), ethyl acetate, acetic acid and ethanolamine. The term "hydrate" refers to the complex where the solvent molecule is water.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

The "pharmaceutically acceptable salt" used in the present invention refers to organic and inorganic salts of the compounds of the present invention. Pharmaceutically acceptable salts are well known in the art. For example, S.

M. Berge et al., describe pharmaceutically acceptable salts in detail in J. Pharmaceutical Sciences, 1977, 66: 1-19, which is incorporated herein by reference. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, malonate, methanesulfonate, 2-naphthalenesulfonate, nicotinate, nitrate, oleate, palmitate, pamoate, pectate, persulfate, 3-phenylpropionate, picrate, pivalate, propionate, stearate, thiocyanate, p-toluenesulfonate, undecanoate, valerate, and the like. Salts derived from appropriate bases include alkali metal, alkaline earth metal, ammonium and $N^{+}(C_{1-4}\ alkyl)_4$ salts. This invention also envisions the quaternization of any basic nitrogen-containing groups of the compounds disclosed herein. Water or oil soluble or dispersable products may be obtained by such quaternization. Representative alkali or alkaline earth metal salts include sodium, lithium, potassium, calcium, magnesium, and the like. Further pharmaceutically acceptable salts further include, when appropriate, nontoxic ammonium, quaternary ammonium, and amine cations formed using counterions such as halide, hydroxide, carboxylate, sulfate, phosphate, nitrate, $C_{1-8}$ sulfonate or aryl sulfonate.

The term "prodrug" used in the present invention refers to a compound that is transformed *in vivo* into a compound of formula (I), (II), (III) or (IV). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic ($C_{1-24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs can be found in the following literature: T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345.

The term "treatment" as used herein refers to both therapeutic and prophylactic treatment. For example, therapeutic treatment includes alleviating or ameliorating the progression, severity, and/or or duration of an immune-mediated condition, or amelioration of one or more symptoms (particularly, one or more identifiable symptoms) of an immune-mediated condition as a result of administering one or more therapies (e.g., one or more therapeutic agents (e.g., compounds and compositions of the invention)). In certain embodiments, therapeutic treatment includes amelioration of at least one measurable physical parameter of an immune-mediated condition. In other embodiments, therapeutic treatment includes inhibiting the progression of an immune-mediated condition physically, for example, by stabilizing discernible symptoms, or physiologically, for example, by stabilizing physical parameters, or both. In other embodiments, therapeutic treatment includes reducing or stabilizing the severity of immune-mediated diseases. The medicament of the invention can be used in the community to treat people already suffering from immune diseases to reduce the severity of symptoms and reduce the number of days they are sick.

**DESCRIPTION OF COMPOUNDS OF THE INVENTION**

The invention provides a class of novel cerebroside compound with immunosuppressive activity, the pharmaceutical composition thereof, and the use thereof in the preparation of drugs for preventing or treating immune-related diseases.

In one aspect, the present invention provides a compound having the structure described in general formula (I), or a solvate, a metabolite, a pharmaceutically acceptable salt or a prodrug thereof,

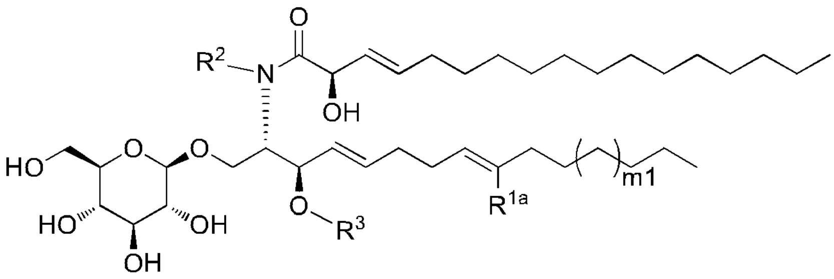

(I),

wherein, m1 is 3, 4 or 6;

$R^{1a}$ is H, $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl;
$R^2$ is H, methyl, ethyl, n-propyl, isopropyl, n-butyl, isobutyl or *tert-butyl;*
$R^3$ is H, $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl.

In some embodiments, $R^{1a}$ is H, methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

In some embodiments, $R^3$ is H, methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

In another aspect, the present invention provides a compound having the structure described in general formula (II), or a solvate, a metabolite, a pharmaceutically acceptable salt or a prodrug thereof,

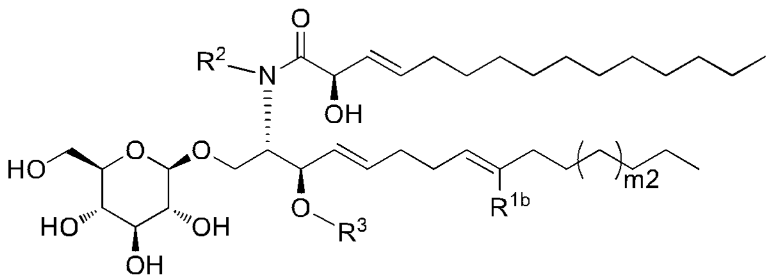

(II),

wherein, m2 is 3, 4, 5 or 6;

$R^{1b}$ is H, $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl;
$R^2$ is H, methyl, ethyl, n-propyl, isopropyl, n-butyl, isobutyl or *tert-butyl;*
$R^3$ is H, $C_{1-6}$ alkyl or $C_{2-6}$ alkenyl, wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl.

In some embodiments, $R^{1b}$ is H, methyl, ethyl, n-propyl, isopropyl, n-butyl, isobutyl, t-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the $C_{1-6}$ alkyl and $C_{2-6}$ alkenyl can be independently and optionally substituted with 1, 2 or 3 substituents selected from F, Cl, Br, I, $CF_3$, CN, cyclopropyl, cyclopentyl or cyclohexyl.

In some embodiments, $R^3$ is H, methyl, ethyl, n-propyl, isopropyl, n-butyl, isobutyl, tert-butyl, $-CH=CH_2$,

$-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CHC(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

In another aspect, the present invention provides a compound having the structure described in general formula (III), or a solvate, a metabolite, a pharmaceutically acceptable salt or a prodrug thereof,

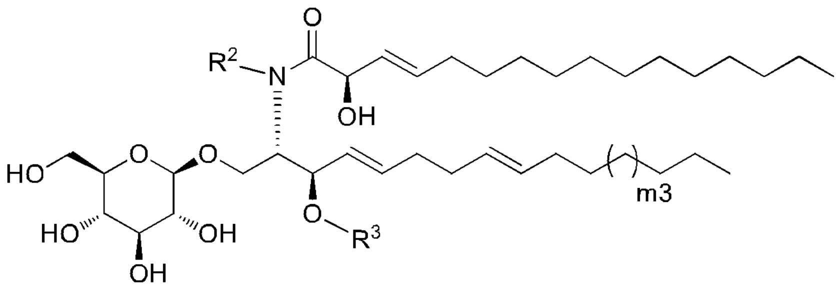

(III),

wherein, m3 is 3, 4, 5 or 6;

$R^2$ is H, methyl, ethyl, n-propyl, isopropyl, n-butyl, isobutyl or *tert-butyl;*

$R^3$ is H, $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl.

In some embodiments, $R^3$ is H, methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein each of methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

In another aspect, the present invention provides a compound having the structure described in general formula (IV), or a solvate, a metabolite, a pharmaceutically acceptable salt or a prodrug thereof,

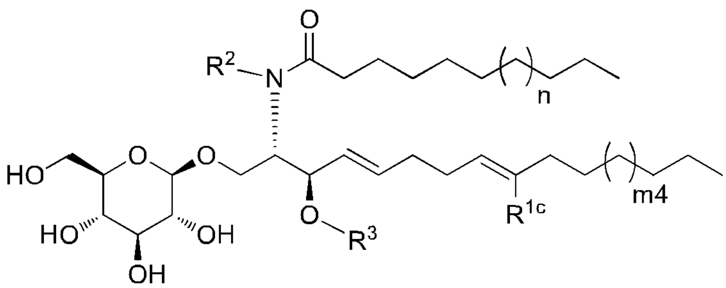

(IV),

wherein,

m4 is 3, 4, 5 or 6;

n is 5, 6 or 7;

$R^{1c}$ is $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl;

$R^2$ is H, methyl, ethyl, *n*-propyl, isopropyl, *n*-butyl, isobutyl or *tert*-butyl;

$R^3$ is H, $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl.

In some embodiments, $R^{1c}$ is methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$,

$-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

In some embodiments, $R^3$ is methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

In some embodiments, the compound of the present invention has any one of the following structures, or a solvate, a metabolite, a pharmaceutically acceptable salt or a prodrug thereof,

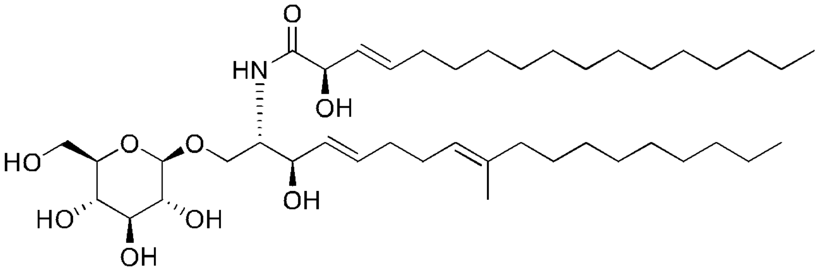

1

,

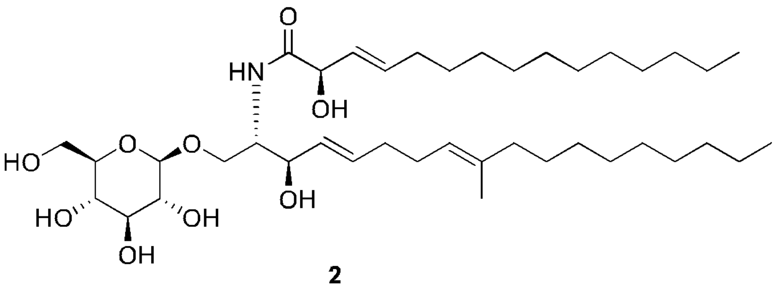

2

,

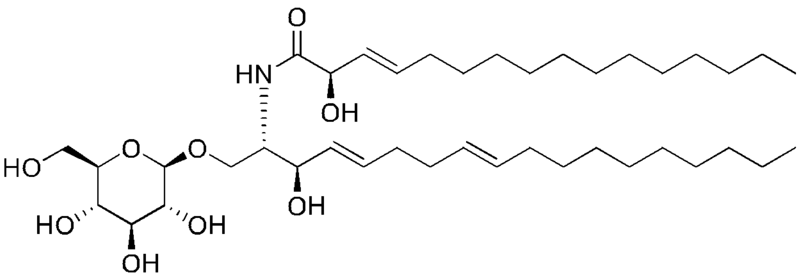

3

,

or

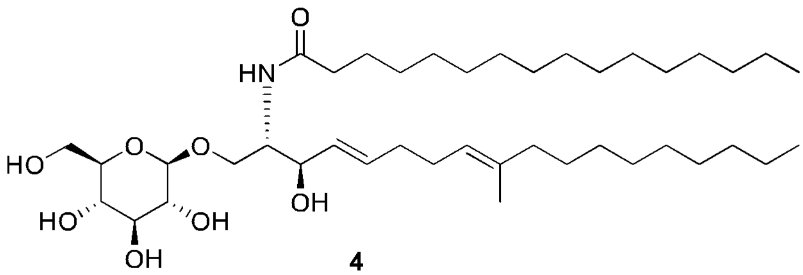

4

.

In some embodiments of the present invention, compounds 1, 2, 3 and 4 of the present invention are extracted from Cordyceps sinensis.

In another aspect, the present invention provides a pharmaceutical composition, which comprises the compound described in the present invention, and a pharmaceutically acceptable adjuvant.

In some embodiments, the pharmaceutical composition of the present invention further comprises other additional therapeutic agent selected from a chemotherapeutic agent or anti-proliferative agent, an anti-inflammatory drug, an immunomodulatory or immunosuppressive agent, a neurotrophic factor, an agent for treating cardiovascular disease, an agent for treating diabetes, and an agent for treating immunodeficiency disorders.

In another aspect, the present invention provides the use of the compound of the present invention and the pharmaceutical composition of the present invention in the manufacture of a medicament for preventing or treating immune-related diseases.

In some embodiments, the immune-related disease is organ transplantation anti-rejection or autoimmune disease, wherein the autoimmune disease is lupus, multiple sclerosis, amyotrophic lateral sclerosis, rheumatoid arthritis, psoriasis, type 1 diabetes, complications from organ transplantation, xeno transplantation, diabetes, asthma, atopic dermatitis, autoimmune thyroid disorders, ulcerative colitis, Crohn's disease, Alzheimer's disease, leukemia or lymphoma.

In another aspect, the present invention provides the use of the compound of the present invention and the pharmaceutical composition of the present invention in the manufacture of a medicament as an immunosuppressant.

In some embodiments, the salt refers to a pharmaceutically acceptable salt. The term "pharmaceutically acceptable" means that the substance or composition must be chemically and/or toxicologically compatible with the other ingredients comprising the preparation and/or with the mammal being treated therewith.

The compounds of the present invention also include other salts of such compounds, which are not necessarily pharmaceutically acceptable salts, and can be used for the preparation and/or purification of the compounds of the present invention and/or for the isolation of the intermediates of enantiomers of the compounds of the present invention.

Pharmaceutically acceptable acid addition salts can be formed with inorganic acids and organic acids, *e.g.,* acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulfate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like.

Any formula given herein is also intended to represent isotopically unlabeled forms as well as isotopically labeled forms of the compounds. Isotopically labeled compounds have structures depicted by the general formulas given herein, except that one or more atoms are replaced by atoms having a selected atomic mass or mass number. Examples of isotopes that can be incorporated into compounds of the invention include isotopes of hydrogen, carbon, nitrogen, oxygen, phosphorous, sulfur, fluorine, and chlorine, such as $^{2}H$, $^{3}H$, $^{11}C$, $^{13}C$, $^{14}C$, $^{15}N$, $^{18}F$, $^{31}P$, $^{32}P$, $^{36}S$, $^{37}Cl$ or $^{125}I$.

**COMPOSITION OF THE COMPOUND OF THE INVENTION, PREPARATIONS AND ADMINISTRATION**

The present invention provides a pharmaceutical composition comprising the compound represented by formula (I), (II), (III) or (IV), or a solvate, a metabolite, a pharmaceutically acceptable salt or a prodrug thereof. The pharmaceutical composition further comprises at least one pharmaceutically acceptable adjuvant, and optionally other therapeutic and/or

prophylactic ingredients. In some embodiments, the pharmaceutical composition comprises an effective amount of at least one pharmaceutically acceptable adjuvant.

As described above, the pharmaceutical composition or the pharmaceutically acceptable composition described in the present invention further comprises pharmaceutically acceptable adjuvants, which, as used herein, include any and all solvents, diluents, liquid vehicles, dispersants or suspension agents, surface active agents, isotonic agents, thickening or emulsifying agents, preservatives, solid binders, lubricants and the like, as suited to the particular dosage form desired. Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York discloses various carriers used in formulating pharmaceutically acceptable compositions and known techniques for the preparation thereof. Except insofar as any conventional carrier medium incompatible with the compounds disclosed herein, such as by producing any undesirable biological effect or otherwise interacting in a deleterious manner with any other components of the pharmaceutically acceptable composition, any other conventional carrier medium and its use are also contemplated by the present invention.

The compounds or compositions of this invention may be administered by any suitable means. The compounds and pharmaceutically acceptable compositions described above may be administered to humans or other animals orally, rectally, parenterally, intracisternally, intravaginally, intraperitoneally, topically (as by powder, ointment or drops), buccally as an oral or nasal spray, etc., depending on the severity of the infection being treated.

The compounds used in the methods of the invention can be formulated in unit dosage form. The term "unit dosage form" refers to physically discrete units suitable as unitary dosages for subjects, each unit containing a predetermined quantity of active material calculated to produce the desired therapeutic effect, optionally in association with suitable pharmaceutical auxiliaries. The unit dosage form can be presented as a single daily dose or one of multiple daily doses (e.g., about 1-4 or more times daily). When multiple daily doses are used, the unit dosage form may be the same or different for each dose.

## USE OF THE COMPOUNDS AND COMPOSITIONS OF THE PRESENT INVENTION

The above compounds and pharmaceutical compositions provided by the present invention can be used to prepare drugs for preventing, treating or alleviating immune-related diseases in patients, preferably, the immune-related disease is organ transplantation anti-rejection or autoimmune disease, preferably, the autoimmune disease is lupus, multiple sclerosis, amyotrophic lateral sclerosis, rheumatoid arthritis, psoriasis, type 1 diabetes, complications from organ transplantation, xeno transplantation, diabetes, asthma, atopic dermatitis, autoimmune thyroid disorders, ulcerative colitis, Crohn's disease, Alzheimer's disease, leukemia or lymphoma, *etc.*

The present invention also provides a method of treating, preventing or alleviating immune-related diseases in a subject, the method comprises administering a therapeutically effective amount of the compound of the present invention or the pharmaceutical composition thereof to a subject. Moreover, the above-mentioned compounds provided by the present invention or their pharmaceutical compositions can be co-administered with other therapies or therapeutic agents. The mode of administration can be carried out simultaneously, sequentially or at certain time intervals. Wherein the other therapy or therapeutic agent is selected from a chemotherapeutic agent or anti-proliferative agent, an anti-inflammatory agent, an immunomodulatory or immunosuppressive agent, a neurotrophic factor, an agent for treating cardiovascular disease, an agent for treating diabetes, or an agent for treating immunodeficiency disorders.

The dosage of the compound or pharmaceutical composition required to implement the effects of treatment, prevention or delay usually depends on the specific compound to be administered, the patient, the specific disease or condition and its severity, the route and frequency of administration, etc., and needs to be determined by the attending physician according to the specific situation. For example, when the compounds or pharmaceutical compositions provided by the present invention are administered by intravenous route, the administration can be performed once a week or even at longer time intervals.

Besides being useful for human treatment, the compounds and pharmaceutical compositions of the present invention are also useful for veterinary treatment of animals such as companion animals, exotic animals and farm animals, including mammals, and the like. In other embodiments, the animals disclosed herein include horses, dogs, and cats. As used herein, the compounds disclosed herein include the pharmaceutically acceptable derivatives thereof.

## DETAILED DESCRIPTION OF PREFERRED EMBODIMENTS

The embodiments of the present invention will be described in detail below. It should be noted that the described embodiments are exemplary and intended to explain the present invention, rather than limiting the present invention.

The solutions of the present invention will be explained below in conjunction with examples. It will be understood by those skilled in the art that the following examples are only used to illustrate the present invention and should not be considered as limiting the scope of the present invention, and other methods for preparing the compounds of the present

invention are considered to be within the scope of the present invention within. If no specific technique or condition is indicated in the examples, it shall be carried out according to the technique or condition described in the literature in this field or according to the product specification. The reagents or instruments used without manufacturer's indication are conventional products that can be purchased from the market.

In some specific embodiments of the present invention, the novel compounds 1, 2, 3 and 4 provided by the present invention are extracted from Cordyceps sinensis.

1. Source of medicinal materials

Cordyceps sinensis comes from YICHANG SHANCHENGSHUIDU CORDYCEPS CO., LTD. in Hubei Province.

2. Experimental Instruments

EYELA Rotary Evaporator N-1001 (Rikakikai Company, Tokyo); Lisui medium and low pressure liquid phase system (Shanghai Lisui Chemical Technology Co., Ltd.); Dionex analytical and preparative high performance liquid chromatography (Thermo Fisher Scientific Company, USA); Phenomenex Gemini $C_{18}$ analytical (4.6 × 250 mm, 5 μm), semi-preparative (10.0 × 250 mm, 5 μm) and preparative (20.0 × 250 mm, 5 μm) chromatographic columns (Phenomenex Company, USA); Phenomenex Biphenyl analytical (4.6 × 250 mm, 5 μm) and semi-preparative (10.0 × 250 mm, 5 μm) chromatographic column (Phenomenex Company, USA) JASCO V-550 UV analyzer was purchased from Jasco International Company, Japan; JASCO FT/IR-480 plus infrared detector was purchased from Jasco International Company, Japan; JASCO P-1020 polarimeter was purchased from Jasco International Company, Japan; Bruker AV-400/600 MHz nuclear magnetic resonance instrument was purchased from Bruker BioSpin, Switzerland; APCI ion source low-resolution mass spectrometer (Bruker Daltonics Company, USA); Waters Synapt G2 TOF high-resolution mass spectrometer was purchased from Waters Company, USA; HERA cell 150i cell incubator was purchased from Thermo Fisher Scientific Company, USA; Model 680 multifunctional microplate reader was purchased from Bio-Rad Laboratories Company, USA.

3. Experimental materials

Analytical pure petroleum ether, cyclohexane, ethyl acetate, and ethanol were purchased from Tianjin Fuyu Fine Chemical Co., Ltd.; deuterated pyridine was purchased from Sigma-Aldrich Company, Germany; chromatography-grade methanol was purchased from Shandong Yuwang Industrial Co., Ltd.;

Silica gel was purchased from Qingdao Ocean Chemical Co., Ltd.; ODS (50 μm) was purchased from YMC Company, Japan; HP-20 macroporous resin was purchased from Mitsubishi Chemical Company, Japan;

Male BABL/C mice were purchased from Hunan Slack Jingda Experimental Animal Co., Ltd.; 96-well plates were purchased from Corning (Shanghai) Management Co., Ltd.; Con A, Hank's solution, and erythrocyte lysate were purchased from Sigma-Aldrich Chemical Company, USA; Cell Counting Kit-8 was purchased from Dojindo Molecular Technologies, Japan.

The following abbreviations are used throughout this specification:

$t_R$: retention time
Pyr-$d_5$: deuterated pyridine
$\delta_H$: chemical shift of hydrogen
$\delta_C$: Compound shift of carbon

If there is no other description, the detection wavelength of chromatographic analysis in the present invention is 205 nm, and the column temperature of the chromatographic column is 30 °C.

Example 1: Separation and preparation of cerebroside fraction (CSE-E7) from Cordyceps sinensis

1.1 Preparation method of CSE-E at ethyl acetate part in Cordyceps sinensis

15 Kg of Cordyceps sinensis was ground, heated and refluxed with 120 L of water to extract for 3 times, each time for 2 h. The mixture was filtered, and the water extraction filtrate was combined to obtain the water extract. The residue was heated and refluxed with 120 L of 95% ethanol to extract for 3 times, each time for 2 h. The mixture was filtered, and the 95% ethanol extraction filtrate was combined to obtain the 95% ethanol extract. The water extract was adsorbed with 20 Kg HP20 macroporous adsorption resin, and then eluted with 250 L of 95% ethanol. The part not absorbed by HP-20 macroporous resin was concentrated under reduced pressure and the obtained extract was marked

as CSW (yield was 27.6%), the 95% ethanol eluted part and the 95% ethanol extract were combined and concentrated under reduced pressure, and the resulting extract was marked as CSE (yield was 20.9%). The CSE part was added with water and stirred, and then extracted successively with cyclohexane and ethyl acetate to obtain CSE-C at the cyclohexane part (extraction yield was 55.0%) and CSE-E at ethyl acetate part (extraction yield was 1.2%).

1.2 Preparation method of cerebroside fraction CSE-E7 fraction from Cordyceps sinensis

CSE-E (38.10 g) was adsorbed and mixed with 50 g of silica gel (100-200 mesh, Qingdao Ocean Chemical Co., Ltd., Qingdao, China), eluted with 280 mL of petroleum ether, and the remaining sample that was still adsorbed by silica gel was evaporated and connected to the ODS column (500 g) (10.0*250 mm) (50 μm, YMC Co. Ltd, Tokyo, Japan), which was separated at medium and low pressure, and eluted with 5%, 10%, 30%, 50%, 70%, 90%, and 100% methanol-water in sequence, with 5 L for each gradient elution. The 100% methanol eluate was combined and concentrated under reduced pressure, and the resulting extract was CSE-E7 (1.4 g).

**Example 2: Separation and identification of monomeric compounds 1, 2, 3, and 4 in the cerebroside fraction CSE-E7 from Cordyceps sinensis**

CSE-E7 (1.4 g) was dissolved in methanol, analyzed by preparative high-performance liquid chromatography, and separated by medium and low pressure ODS column chromatography (3.1 × 20.0 cm, Japan YMC Company). The mobile phase was eluted with a gradient of 85%-100% (v/v) methanol-water for 3 hours, and at the same time, the eluent was connected with a graduated centrifuge tube, about 30 mL into one tube, and every 15 tubes were combined into one fraction. A total of 7 sub-fractions were obtained, namely F7.1, F7.2, F7.3, F7.4, F7.5, F7.6, F7.7. Fraction F7.4 (10.8 mg) was separated by a semi-preparative chromatographic column Phenomenex Biphenyl (10.0 × 250 mm, 5 μm) and eluted isocratically with a mobile phase of 85% methanol-water (v/v) at a flow rate of 3 mL/min to obtain compound 2 ($t_R$: 43.8 min, 3.5 mg). Fraction F7.5 (93.8 mg) was separated by a semi-preparative chromatographic column Phenomenex Gemini C18 (10.0 × 250 mm, 5 μm) and eluted isocratically with a mobile phase of 92% methanol-water (v/v) at a flow rate of 3 mL/min to obtain compound 3 ($t_R$: 51.6 min, 4.1 mg). Fraction F7.6 (523.4 mg) was separated by a preparative chromatographic column Phenomenex Gemini C18 (20.0 × 250 mm, 5 μm) and eluted isocratically with a mobile phase of 93% methanol-water (v/v) at a flow rate of 8 mL/min to obtain fraction F7.6.1 ($t_R$: 50.0~55.0 min), F7.6.2 ($t_R$: 60.0~65.0 min), F7.6.3 ($t_R$: 66.0~70.0 min) and F7.6.4 ($t_R$: 74.0~78.0 min). Fraction F7.6.2 (20.7 mg) was separated by a semi-preparative chromatographic column Phenomenex Biphenyl (10.0 × 250 mm, 5 μm) and eluted isocratically with a mobile phase of 87% methanol-water (v/v) at a flow rate of 3 mL/min to obtain compound 1 ($t_R$: 31.6 min, 9.3 mg). Fraction F7.6.3 (8.8 mg) was separated by a semi-preparative chromatographic column Phenomenex Biphenyl (10.0 × 250 mm, 5 μm) and eluted isocratically with a mobile phase of 87% methanol-water (v/v) at a flow rate of 3 mL/min to obtain compound 4 ($t_R$: 29.2 min, 2.5 mg).

The structures and identification data of compounds 1, 2, 3 and 4 are as follows:

**Compound 1:**

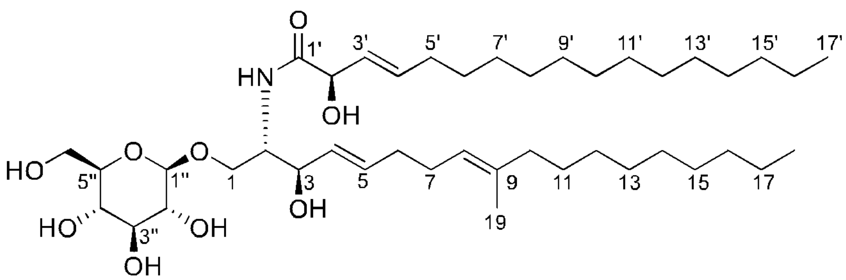

Compound 1: white powder; [α]27 D +50.5 (c 0.40, MeOH); UV (MeOH) $\lambda_{max}$ (log $\varepsilon$) 202 (4.22) nm, IR (KBr) $\nu_{max}$ 3283, 2921, 1639, 1531, 1466, 1081, 1029, 968 $cm^{-1}$; APCI-MS/MS (positive) *m/z* 740, 722, 560, 294, 276; HRESIMS (positive) *m/z* 740.5668 $[M + H]^+$ (calcd. for $C_{42}H_{78}NO_9$, 740.5671); $^1H$ and $^{13}C$ NMR are shown in Table 1.

**Compound 2:**

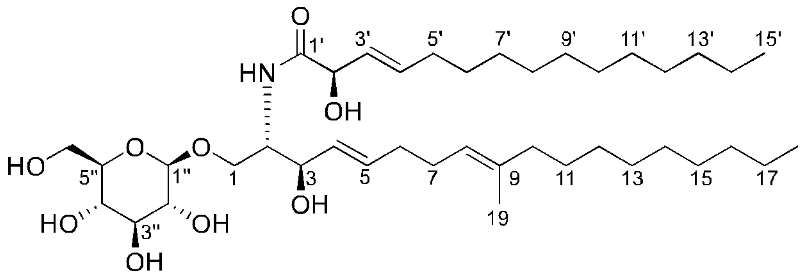

Compound 2: white powder; [α]27 D +43.4 (c 0.60, MeOH); UV (MeOH) $\lambda_{max}$ (log $\varepsilon$) 203 (4.09) nm; IR (KBr) $\nu_{max}$ 3332, 2915, 1659, 1539, 1466, 1073 $cm^{-1}$; APCI-MS/MS (positive) *m/z* 712, 694, 532, 294, 276; HRESIMS (positive) *m/z* 712.5357 $[M + H]^+$ (calcd. for $C_{40}H_{74}NO_9$, 712.5358); $^1H$ and $^{13}C$ NMR are shown in Table 1.

**Compound 3:**

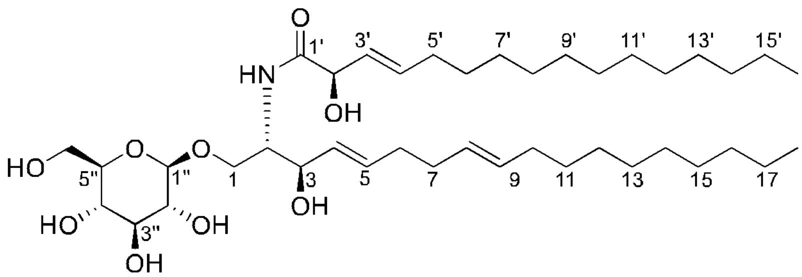

Compound 3: white powder; [α]27 D +33.1 (*c* 0.60, MeOH); UV (MeOH) $\lambda_{max}$ (log $\varepsilon$) 202 (4.02) nm; IR (KBr) $\nu_{max}$ 3218, 2921, 1639, 1539, 1466, 1078 $cm^{-1}$; APCI-MS/MS (positive) *m/z* 712, 694, 532, 280, 262; HRESIMS (positive) *m/z* 712.5354 $[M + H]^+$ (calcd. for $C_{40}H_{74}NO_9$, 712.5358); $^1H$ and $^{13}C$ NMR are shown in Table 2.

**Compound 4:**

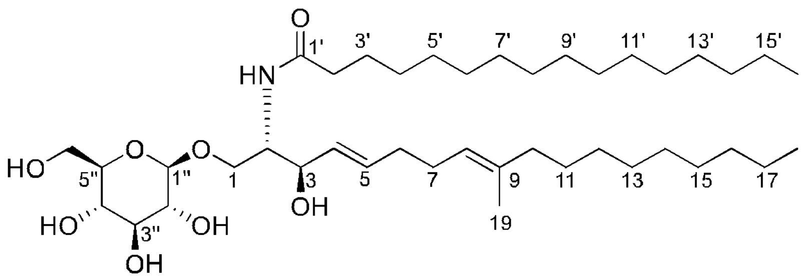

Compound **4:** white powder; [α]27 D +80.3 (c 0.30, MeOH); UV (MeOH) $\lambda_{max}$ (log $\varepsilon$) 202 (3.36) nm; IR (KBr) $\nu_{max}$ 3172, 2918, 1720, 1542, 1466, 1079 $cm^{-1}$; APCI-MS/MS (positive) *m/z* 712, 694, 532, 294, 276; HRESIMS (positive) *m/z* 712.5721 $[M + H]^+$ (calcd. for $C_{41}H_{78}NO_8$, 712.5722); $^1H$ and $^{13}C$ NMR are shown in Table 2.

**Table 1** NMR data attribution of compounds 1 and 2 (600 MHz, in Pyr-$d_5$)

| No. | Compound **1** | | Compound **2** | |
|---|---|---|---|---|
| | $\delta_C$, mult. | $\delta_H$ (*J* in Hz) | $\delta_C$, mult. | $\delta_H$ (*J* in Hz) |
| | Long chain base | | Long chain base | |
| 1 | 70.0, $CH_2$ | 4.73, dd (10.5, 5.8), Ha | 69.8, $CH_2$ | 4.74, dd (10.3, 5.6), Ha |
| | | 4.26, dd (10.5, 3.7), Hb | 4.26, | 4.26, dd (10.3, 3.6), Hb |
| 2 | 54.7, CH | 4.78 | 54.4, CH | 4.79 |
| 3 | 72.3, CH | 4.76, dd (5.7, 1.6) | 72.2, CH | 4.77, dd (5.6, 1.8) |

(continued)

| No. | Compound **1** | | Compound **2** | |
|---|---|---|---|---|
| | $\delta_C$, mult. | $\delta_H$ (*J* in Hz) | $\delta_C$, mult. | $\delta_H$ (*J* in Hz) |
| | Long chain base | | Long chain base | |
| 4 | 131.8, CH | 6.00, dd (15.4, 5.7) | 131.6, CH | 6.00, dd (15.5, 5.6) |
| 5 | 132.3, CH | 5.94, dt (15.4, 4.8) | 132.3, CH | 5.94, dt (15.5, 4.8) |
| 6 | 33.0, $CH_2$ | 2.16 | 32.9, $CH_2$ | 2.16 |
| 7 | 28.2, $CH_2$ | 2.16 | 28.1, $CH_2$ | 2.16 |
| 8 | 124.2, CH | 5.27, t (5.4) | 124.0, CH | 5.27, t (5.4) |
| 9 | 135.5, C | - | 135.6, C | - |
| 10 | 40.0, $CH_2$ | 2.02, t (7.3) | 39.8, $CH_2$ | 2.02, t (7.3) |
| 11 | 28.3, $CH_2$ | 1.42, quint (7.3) | 28.2, $CH_2$ | 1.42, quint (7.1) |
| 12 | 29.6*, $CH_2$ | 1.26 | 29.5*, $CH_2$ | 1.26 |
| 13 | 29.6*, $CH_2$ | 1.26 | 29.5*, $CH_2$ | 1.26 |
| 14 | 29.6*, $CH_2$ | 1.26 | 29.5*, $CH_2$ | 1.26 |
| 15 | 29.6*, $CH_2$ | 1.26 | 29.5*, $CH_2$ | 1.26 |
| 16 | 32.1, $CH_2$ | 1.26 | 32.0, $CH_2$ | 1.26 |
| 17 | 22.9, $CH_2$ | 1.26 | 22.8, $CH_2$ | 1.26 |
| 18 | 14.3, $CH_3$ | 0.87, t (6.4) | 14.2, $CH_3$ | 0.87, t (6.4) |
| 19 | 16.1, $CH_3$ | 1.63, s | 16.0, $CH_3$ | 1.63, s |
| 2-NH | | 8.37, d (8.5) | | 8.37, d (8.6) |
| | Long chain acyl | | Long chain acyl | |
| 1’ | 173.8, C | - | 173.9, C | - |
| 2’ | 73.5, CH | 5.12, d (5.1) | 73.4, CH | 5.12, d (4.3) |
| 3’ | 130.0, CH | 6.14, dd (15.5, 5.1) | 129.8, CH | 6.13, dd (15.0, 4.3) |
| 4’ | 132.3, CH | 6.19, dt (15.5, 6.2) | 132.3, CH | 6.20, dt (15.0, 6.2) |
| 5’ | 32.7, $CH_2$ | 2.11 | 32.6, $CH_2$ | 2.11 |
| 6’ | 29.6, $CH_2$ | 1.38, quint (7.2) | 29.5, $CH_2$ | 1.38, quint (7.1) |
| 7’ | 29.9*, $CH_2$ | 1.26 | 29.8*, $CH_2$ | 1.26 |
| 8’ | 29.9*, $CH_2$ | 1.26 | 29.8*, $CH_2$ | 1.26 |
| 9’ | 29.9*, $CH_2$ | 1.26 | 29.8*, $CH_2$ | 1.26 |
| 10’ | 29.9*, $CH_2$ | 1.26 | 29.8*, $CH_2$ | 1.26 |
| 11’ | 29.9*, $CH_2$ | 1.26 | 29.8*, $CH_2$ | 1.26 |
| 12’ | 29.9*, $CH_2$ | 1.26 | 29.8*, $CH_2$ | 1.26 |
| 13’ | 29.9*, $CH_2$ | 1.26 | 32.0, $CH_2$ | 1.26 |
| 14’ | 29.9*, $CH_2$ | 1.26 | 22.8, $CH_2$ | 1.26 |
| 15’ | 32.1, $CH_2$ | 1.26 | 14.2, $CH_3$ | 0.87, t (6.4) |
| 16’ | 22.9, $CH_2$ | 1.26 | - | - |
| 17’ | 14.3, $CH_3$ | 0.87, t (6.4) | - | - |

(continued)

| | Glycosyl | | Glycosyl | |
|---|---|---|---|---|
| 1" | 105.6, CH | 4.93, d (7.8) | 105.3, CH | 4.93, d (8.0) |
| 2" | 75.1, CH | 4.05, t (7.8) | 74.9, CH | 4.05, t (8.0) |
| 3" | 78.4, CH | 4.24 | 78.4, CH | 4.24 |
| 4" | 71.5, CH | 4.24 | 71.4, CH | 4.24 |
| 5" | 78.6, CH | 3.92 | 78.4, CH | 3.93 |
| 6" | 62.6, $CH_2$ | 4.52, dd (11.8, 2.1), Ha | 62.5, $CH_2$ | 4.52, dd (11.4, 2.3), Ha |
| | | 4.37, dd (11.8, 5.1), Hb | 4.38, | 4.38, dd (11.8, 5.0), Hb |
| *signal attribution may be interchanged within the same column | | | | |

**Table 2** NMR data attribution of compounds 3 and 4 (600 MHz, in Pyr-$d_5$)

| No. | Compound **3** | | Compound **4** | |
|---|---|---|---|---|
| | $\delta_C$, mult. | $\delta_H$ ($J$ in Hz) | $\delta_C$, mult. | $\delta_H$ ($J$ in Hz) |
| | Long chain base | | Long chain base | |
| 1 | 69.8, $CH_2$ | 4.74, dd (10.7, 6.0), Ha | 70.5, $CH_2$ | 4.78, dd (10.6, 5.3), Ha |
| | | 4.23, brd (10.7), Hb | | 4.25, dd (10.6, 3.1), Hb |
| 2 | 54.4, CH | 4.77 | 55.1, CH | 4.84 |
| 3 | 72.1, CH | 4.72, dd (5.9, 1.6) | 72.7, CH | 4.80, brd (6.2) |
| 4 | 131.7, CH | 5.96, dd (15.8, 5.9) | 132.2, CH | 6.06, dd (15.3, 6.4) |
| 5 | 132.0, CH | 5.89, dt (15.8, 4.9) | 132.3, CH | 5.97, dt (15.3, 6.6) |
| 6 | 32.7, $CH_2$ | 2.13 | 33.1, $CH_2$ | 2.15 |
| 7 | 32.6, $CH_2$ | 2.13 | 28.3, $CH_2$ | 2.15 |
| 8 | 129.8, CH | 5.46, dt (15.7, 5.7) | 124.1, CH | 5.26, t (5.6) |
| 9 | 130.9, CH | 5.50, dt (15.7, 5.2) | 135.7, C | - |
| 10 | 32.8, $CH_2$ | 2.00 | 40.0, $CH_2$ | 2.01, t (7.0) |
| 11 | 29.5, $CH_2$ | 1.35, quint (7.1) | 28.2, $CH_2$ | 1.42, quint (7.0) |
| 12 | 29.5*, $CH_2$ | 1.24 | 29.6*, $CH_2$ | 1.27 |
| 13 | 29.5*, $CH_2$ | 1.24 | 29.6*, $CH_2$ | 1.27 |
| 14 | 29.5*, $CH_2$ | 1.24 | 29.6*, $CH_2$ | 1.27 |
| 15 | 29.5*, $CH_2$ | 1.24 | 29.6*, $CH_2$ | 1.27 |
| 16 | 32.0, $CH_2$ | 1.24 | 32.1, $CH_2$ | 1.27 |
| 17 | 22.8, $CH_2$ | 1.24 | 22.9, $CH_2$ | 1.27 |
| 18 | 14.2, $CH_3$ | 0.85, t (6.7) | 14.3, $CH_3$ | 0.87, t (6.4) |
| 19 | - | - | 16.1, $CH_3$ | 1.62, s |
| 2-NH | | 8.36, d (8.6) | | 8.39, d (8.4) |
| | Long chain acyl | | Long chain acyl | |
| 1’ | 173.9, C | - | 173.4, C | - |
| 2’ | 73.4, CH | 5.11, d (4.9) | 36.9, $CH_2$ | 2.44, t (7.5) |

(continued)

| | Long chain acyl | | Long chain acyl | |
|---|---|---|---|---|
| 3' | 129.9, CH | 6.10, dd (15.5, 5.0) | 26.4, $CH_2$ | 1.83 |
| 4' | 132.2, CH | 6.19, dt (15.5, 6.6) | 29.6, $CH_2$ | 1.37 |
| 5' | 32.6, $CH_2$ | 2.09 | 29.9*, $CH_2$ | 1.27 |
| 6' | 29.5, $CH_2$ | 1.35, quint (7.1) | 29.9*, $CH_2$ | 1.27 |
| 7' | 29.8*, $CH_2$ | 1.24 | 29.9*, $CH_2$ | 1.27 |
| 8' | 29.8*, $CH_2$ | 1.24 | 29.9*, $CH_2$ | 1.27 |
| 9' | 29.8*, $CH_2$ | 1.24 | 29.9*, $CH_2$ | 1.27 |
| 10' | 29.8*, $CH_2$ | 1.24 | 29.9*, $CH_2$ | 1.27 |
| 11' | 29.8*, $CH_2$ | 1.24 | 29.9*, $CH_2$ | 1.27 |
| 12' | 29.8*, $CH_2$ | 1.24 | 29.9*, $CH_2$ | 1.27 |
| 13' | 29.8*, $CH_2$ | 1.24 | 29.9*, $CH_2$ | 1.27 |
| 14' | 32.0, $CH_2$ | 1.24 | 32.1, $CH_2$ | 1.27 |
| 15' | 22.8, $CH_2$ | 1.24 | 22.9, $CH_2$ | 1.27 |
| 16' | 14.2, $CH_3$ | 0.85, t (6.7) | 14.3, $CH_3$ | 0.87, t (6.4) |
| | Glycosyl | | Glycosyl | |
| 1" | 105.4, CH | 4.90, d (7.7) | 105.9, CH | 4.92, d (7.8) |
| 2" | 75.0, CH | 4.03, t (7.7) | 75.3, CH | 4.08, t (7.8) |
| 3" | 78.3, CH | 4.22 | 78.6, CH | 4.26 |
| 4" | 71.4, CH | 4.20 | 71.6, CH | 4.26 |
| 5" | 78.4, CH | 3.90 | 78.6, CH | 3.96 |
| 6" | 62.5, $CH_2$ | 4.50, brd (11.8), Ha | 62.7, $CH_2$ | 4.52, brd (11.4), Ha |
| | | 4.33, dd (11.8, 5.3), Hb | | 4.37, dd (11.4, 4.2), Hb |
| *signal attribution may be interchanged within the same column | | | | |

**Example 3: the immunosuppressive activity test of compounds 1,2,3,4**

**3.1 Solution preparation**

Medium: 89% medium + 10% fetal bovine serum + 1% double antibody, stored at 2-8 °C.

PBS: 1 pack of PBS was dissolved completely with 1L of pure water, subpackaged and sterilized at high temperature.

10% CCK-8: 1 mL of CCK8 was added to 9 mL of culture medium to prepare 10% CCK-8 detection solution, which was prepared and used immediately.

ConA: 5 mg of ConA was accurately weighed and added with 1 mL of sterile PBS to fully dissolve to prepare a 5 mg/mL mother solution, which was subpackaged into 50 μL per tube and stored at -20 °C. When in use, after thawing, complete medium was used to prepare a working solution with a concentration of 150 μg/mL.

**3.2 Experimental method**

**3.2.1 Cell acquisition:** BABL/C male mice were sacrificed after eyeball extraction and bloodletting, soaked in 75% ethanol for 10 min, and the spleen was aseptically removed in an ultra-clean workbench, and placed in a petri dish filled with PBS. A syringe was used to absorb PBS to rinse the surface and interior of the spleen until the spleen turned white, then the spleen was gently torn up with tweezers. The spleen was gently crushed with a 5 mL syringe on the steel

mesh to make a single-cell suspension, which was filtered through a 200-mesh sieve. The filtrate was centrifuged for 10 min (1000 rpm/min); after centrifugation, the supernatant was discarded. After the cell pellet was resuspended with PBS, the mixture was centrifuged again for 10 min (1000 rpm/min), and the operation was repeated once; 2 mL of erythrocyte lysate was added to the cell pellet. After the mixture was mixed evenly and allowed to stand for 5 min, 8 mL of PBS was added to mix evenly, and then the mixture was centrifuged at 1000 rpm/min for 5 min. After centrifugation, the supernatant was discarded, the cell pellet was resuspended with PBS, then the mixture was centrifuged again for 10 min (1000 rpm/min), and the operation was repeated once; after centrifugation, the supernatant was discarded, the cell pellet was suspended in 1 mL of RPMI-1640 complete medium (containing 10% fetal bovine serum and 1% double antibody). 2-3 mL medium was added to dilute. A cell counter was used for cell counting (new cell type), and complete medium was used to adjust the cell concentration to $2*10^6$ cells/mL.

**3.2.2 Sample preparation:** appropriate amount of compounds 1, 2, 3, and 4 were dissolved in DMSO respectively to prepare 64 mM mother solution, and then diluted with medium to obtain solutions with a concentration of 128 μM respectively, and then gradiently diluted with medium to obtain sample dilutions with concentrations of 64 μM, 32 μM, 16 μM, 8 μM, and 4 μM, respectively.

**3.2.3 Detection of cell viability:** the prepared cell suspension was added to a 96-well plate. The seeding cell density was 2*105 cells/well, and the added cell volume was 100 μL. Compounds 1, 2, 3, and 4 were added at 100 μL/well, with 5 replicate wells for each concentration, and then incubated in a 37°C, 5% $CO_2$ incubator for 60 h, and then incubated in a 37°C incubator for 4-5 hours with serum-free medium and 10% CCK-8. The absorbance (A) value of each well was detected at 450 nm by a microplate reader. A control group was set up: cells + 1640 medium. The results showed that compared with the control group, compounds 1, 2, 3, and 4 showed no cytotoxic effect on spleen lymphocytes at various concentrations (64, 32, 16, 8, 4, and 2 μM).

**3.2.4 Detection of cell proliferation inhibition:** the prepared cell suspension was added to the 96-well plate. The seeding cell density was $2*10^5$ cells/well, and the added cell volume was 100 μL. Samples were added at 100 μL/well, with 5 replicate wells for each concentration, and then incubated in a 37°C, 5% $CO_2$ incubator for 12 h. 10 μL of the supernatant were carefully suck out. 10 μL ConA with a concentration of 150 μg/mL (final concentration: 7.5 μg/mL) was added to the corresponding group, and then the mixture was incubated in a 37 °C, 5% $CO_2$ incubator for 48 h, and finally incubated in a 37°C incubator for 4-5 hours with serum-free medium and 10% CCK-8. The absorbance (A) value of each well was detected at 450 nm by a microplate reader. A negative control group and a model control group were set up, which were cells + 1640 medium, cells + ConA + 1640 medium, respectively.

**3.2.5 Data processing:** the proliferation rate (viability) of the sample to spleen lymphocytes was calculated according to the following formula:

$$\text{Proliferation rate\%} = [\text{As}/\text{Ac}] \times 100\%$$

As: Absorbance of sample group (spleen lymphocytes + samples to be tested + CCK-8)
Ac: Absorbance of control group (spleen lymphocytes + CCK-8)

The proliferation inhibition rate of the sample to spleen lymphocytes was calculated according to the following formula:

$$\text{Proliferation inhibition rate \%} = [(\text{Am-As})/(\text{Am-Ac})] \times 100\%$$

Am: Absorbance of model control group (spleen lymphocytes + ConA + CCK-8)
As: Absorbance of sample group (spleen lymphocytes + samples to be tested + ConA + CCK-8)
Ac: Absorbance of negative control group (spleen lymphocytes + CCK-8)

### 3.3 Experimental results

The experimental results of compounds **1, 2, 3, 4** on the cell proliferation inhibition rate of Con A-induced spleen lymphocyte proliferation are shown in Table 3 below; according to the log value of the concentration, linear fitting was performed with the log value x and the inhibition rate y, the fitted curve could calculate the $IC_{50}$, and the $IC_{50}$ values corresponding to compounds **1, 2, 3,** and **4** were 4.0, 10.4, 19.5, and 1.5 μM, respectively.

Table 3 Inhibitory rate (%) of compounds **1-4** on the proliferation of spleen lymphocytes induced by Con A

| Compound \ Concentration (μM) | 64 | 32 | 16 | 8 | 4 | 2 |
|---|---|---|---|---|---|---|
| **1** | 152.4±27.2*** | 147.6±27.6*** | 150.4±29.7*** | 145.8±42.3*** | 136.1±51.0*** | 129.7±45.5*** |
| **2** | 140.2±8.0*** | 126.4±8.8*** | 100.4±6.4*** | 77.7±12.1*** | 54.3±11.5** | 43.7±12.7* |
| **3** | 123.2±29.6*** | 84.2±44.4** | 52.0±8.9** | 37.4±17.8* | 30.9±2.0* | 4.5±21.7 |
| **4** | 120.5±61.2** | 119.9±66.9** | 102.1±64.3** | 100.2±78.0* | 70.7±82.9 | 72.2±64.3* |

***: Compared with the model group, $p < 0.001$; **: Compared with the model group, $p < 0.01$; *: Compared with the model group, $p < 0.05$

It can be seen from Table 3 that the compounds of the present invention all show good immunosuppressive activity in inhibiting Con A-induced proliferation of mouse splenocytes.

Reference throughout this specification to "an embodiment", "some embodiments", "one embodiment", "another example", "an example", "a specific example", or "some examples" means that a particular feature, structure, material, or characteristic described in connection with the embodiment or example is included in at least one embodiment or example of the present disclosure. In this specification, the schematic representations of the above terms are not necessarily directed to the same embodiment or example. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

The above is only preferred embodiments of the present invention, it should be pointed out that for those of ordinary skill in the art, without departing from the concept of the present invention, some improvements and modifications can also be made, and these improvements and modifications should also be considered within the protection scope of the present invention.

## Claims

1. A compound having the structure described in general formula (I), (II), (III) or (IV), or a solvate, a metabolite, a pharmaceutically acceptable salt or a prodrug thereof,

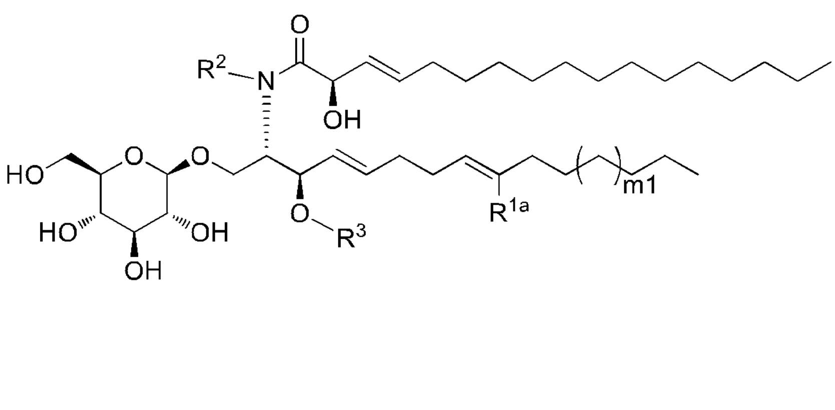

(I),

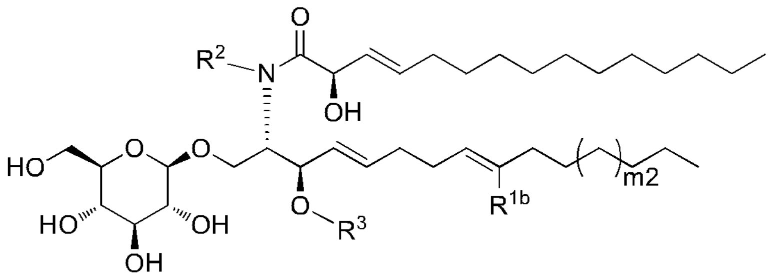

(II),

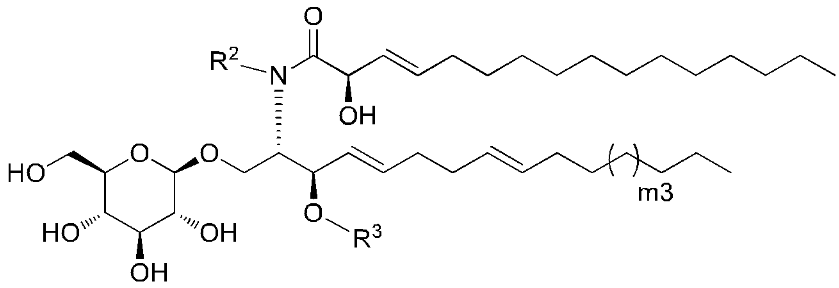

(III),

or

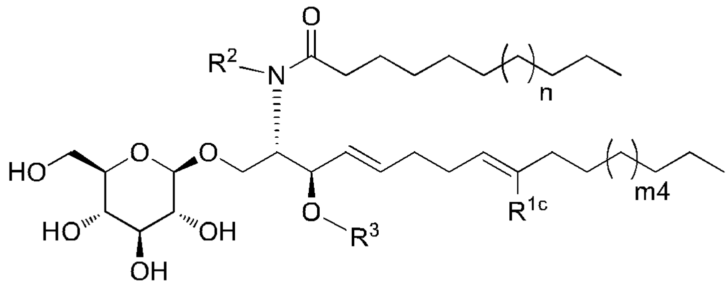

(IV),

wherein,

m1 is 3, 4 or 6;
each of m2, m3 and m4 is independently 3, 4, 5 or 6;
n is 5, 6 or 7;
each of $R^{1a}$ and $R^{1b}$ is independently H, $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl;
$R^{1c}$ is $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl;
each $R^2$ is independently H, methyl, ethyl, n-propyl, isopropyl, n-butyl, isobutyl or *tert-butyl;*
each $R^3$ is independently H, $C_{1-6}$ alkyl or $C_{2-6}$ alkenyl; wherein the $C_{1-6}$ alkyl and $C_{2-6}$ alkenyl can be independently and optionally substituted with 1, 2 or 3 substituents selected from F, Cl, Br, I, $CF_3$, CN or $C_{3-6}$ cycloalkyl.

**2.** The compound of claim 1, wherein,
each of $R^{1a}$ and $R^{1b}$ is independently H, methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

**3.** The compound of claim 1, wherein,

$R^{1c}$ is methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

**4.** The compound of claim 1, wherein,
each $R^3$ is independently H, methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ or $-CH_2C(CH_3)=C(CH_3)_2$; wherein the methyl, ethyl, n-propyl, isopropyl, n-butyl, isobutyl, tert-butyl, $-CH=CH_2$, $-CH_2CH=CH_2$, $-CH_2C(CH_3)=CH_2$, $-CH_2CH=CH(CH_3)$, $-CH_2CH=C(CH_3)_2$, $-CHCH_3CH=C(CH_3)_2$ and $-CH_2C(CH_3)=C(CH_3)_2$ can be independently and optionally substituted with 1, 2 or 3 substituents selected from F, Cl, Br, I, $CF_3$, CN, cyclopropyl, cyclopentyl or cyclohexyl.

**5.** The compound of any one of claims 1-4, having any one of the following structures, or a solvate, a metabolite, a pharmaceutically acceptable salt or a prodrug thereof,

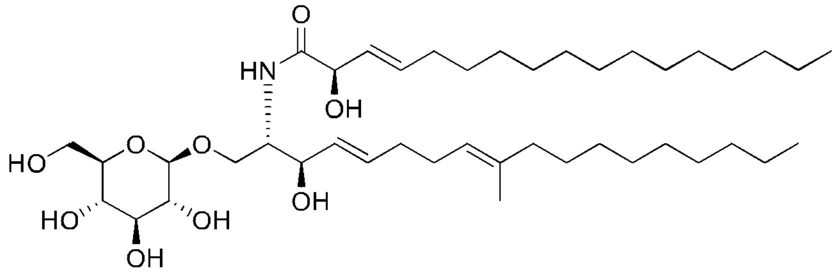

**1**

,

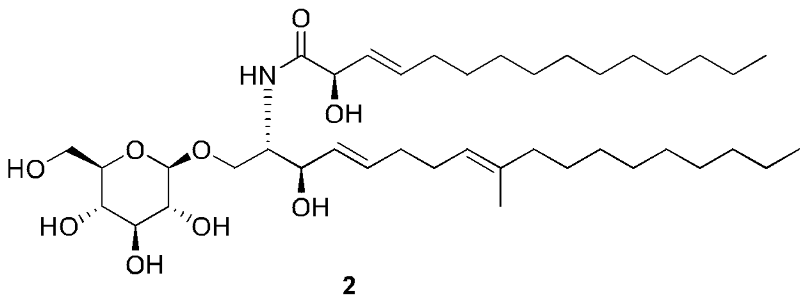

**2**

,

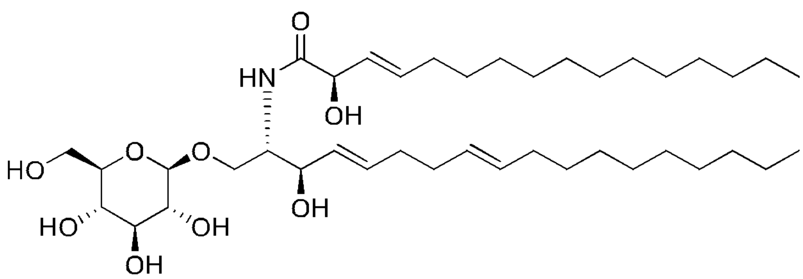

**3**

,

or

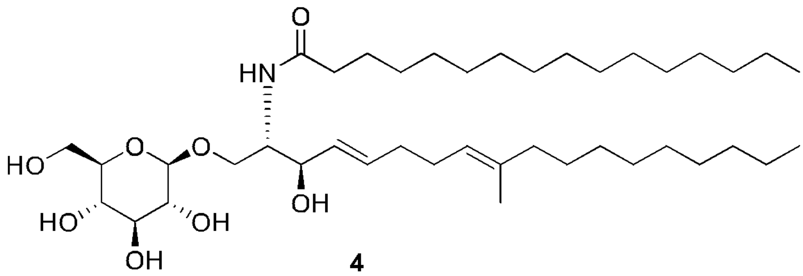

4

.

6. A pharmaceutical composition, comprising the compound of any one of claims 1-5, and a pharmaceutically acceptable adjuvant.

7. The pharmaceutical composition of claim 6, further comprising other additional therapeutic agent selected from a chemotherapeutic agent or anti-proliferative agent, an anti-inflammatory drug, an immunomodulatory or immunosuppressive agent, a neurotrophic factor, an agent for treating cardiovascular disease, an agent for treating diabetes, and an agent for treating immunodeficiency disorders.

8. Use of the compound of any one of claims 1-5 or the pharmaceutical composition of claim 6 or 7 in the manufacture of a medicament for preventing or treating immune-related diseases.

9. The use of claim 8, wherein the immune-related disease is organ transplantation anti-rejection or autoimmune disease, wherein the autoimmune disease is lupus, multiple sclerosis, amyotrophic lateral sclerosis, rheumatoid arthritis, psoriasis, complications from organ transplantation, xeno transplantation, diabetes, asthma, atopic dermatitis, autoimmune thyroid disorders, ulcerative colitis, Crohn’s disease, Alzheimer’s disease, leukemia or lymphoma.

10. Use of the compound of any one of claims 1-5 or the pharmaceutical composition of claim 6 or 7 in the manufacture of a medicament as an immunosuppressant.

## INTERNATIONAL SEARCH REPORT

International application No.
**PCT/CN2021/085308**

### A. CLASSIFICATION OF SUBJECT MATTER

A61K 31/7032(2006.01)i; A61P 37/02(2006.01)i; A61P 37/00(2006.01)i; C07H 15/10(2006.01)i; C07H 1/08(2006.01)i

According to International Patent Classification (IPC) or to both national classification and IPC

### B. FIELDS SEARCHED

Minimum documentation searched (classification system followed by classification symbols)

A61K, A61P, C07H

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, STN, CNKI, Web of science: 脑苷脂, 葡萄糖脑苷脂, 葡糖脑苷脂, 糖鞘脂, 糖基鞘脂, 冬虫夏草, 虫草, 免疫, cerebroside, glycoceramide, GlcCer, sphingolipids, glycosphingolipids, Cordyceps sinensis, stn claims 1, 5化合物structural formula search

### C. DOCUMENTS CONSIDERED TO BE RELEVANT

| Category* | Citation of document, with indication, where appropriate, of the relevant passages | Relevant to claim No. |
|---|---|---|
| X | 刘瑞全 (LIU, Ruiquan). "脑苷脂Chrysogeside B及其类似物的合成、生物活性研究 (Synthesis and Biological Study of Chrysogeside B and Its Variants)" *《中国优秀博硕士学位论文全文数据库（博士）工程科技I辑》 (Chinese Selected Doctoral Dissertations and Master's Theses Full-Text Databases (Doctoral), Engineering Science & Technology I)*, No. 5, 15 May 2020 (2020-05-15), ISSN: 1674-022X, page 6 figures 1-4（B）, page 9 lines 10-17 | 1-10 |
| X | JP 2012224564 A (NATIONAL UNIVERSITY CORPORATION TOKYO UNIVERSITY OF AGRICULTURE AND TECHNOLOGY) 15 November 2012 (2012-11-15) claim 4 | 1-7 |
| X | Kelly.M.Jenkins et al. "Thraustochytrosides A-C: new glycosphingolipids from a unique marine protist, Thraustochytrium globosum" *Tetrahedron Letters,* Vol. 40, No. 43, 22 October 1999 (1999-10-22), ISSN: 0040-4039, page 7638 compound 2 | 1-7 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 November 2021** | **03 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/085308**

**C. DOCUMENTS CONSIDERED TO BE RELEVANT**

| Category* | Citation of document, with indication, where appropriate, of the relevant passages | Relevant to claim No. |
|---|---|---|
| Y | 刘瑞全 (LIU, Ruiquan). "脑苷脂Chrysogeside B及其类似物的合成、生物活性研究 (Synthesis and Biological Study of Chrysogeside B and Its Variants)" *《中国优秀博硕士学位论文全文数据库（博士） 工程科技I辑》 (Chinese Selected Doctoral Dissertations and Master's Theses Full-Text Databases (Doctoral), Engineering Science & Technology I),* No. 5, 15 May 2020 (2020-05-15), ISSN: 1674-022X, page 6 figures 1-4（B）, page 9 lines 10-17 | 8-10 |
| Y | JP 2012224564 A (NATIONAL UNIVERSITY CORPORATION TOKYO UNIVERSITY OF AGRICULTURE AND TECHNOLOGY) 15 November 2012 (2012-11-15) claim 4 | 8-10 |
| Y | Kelly.M.Jenkins et al. "Thraustochytrosides A-C: new glycosphingolipids from a unique marine protist, Thraustochytrium globosum" *Tetrahedron Letters,* Vol. 40, No. 43, 22 October 1999 (1999-10-22), ISSN: 0040-4039, page 7638 compound 2 | 8-10, |
| A | CN 101123986 A (ENZO THERAPEUTICS INC.) 13 February 2008 (2008-02-13) entire document | 1-10 |
| A | 桑已曙 等 (SANg, Yishu et al.). "脑苷脂类化合物研究进展 (Research Progress of Cerebrosides)" *《中国生化药物杂志》 (Chinese Journal of Biochemical Pharmaceutics),* Vol. 21, No. 4, 30 August 2000 (2000-08-30), ISSN: 1005-1678, pp. 211-213 | 1-10 |

Form PCT/ISA/210 (second sheet) (January 2015)

**INTERNATIONAL SEARCH REPORT**

International application No.
**PCT/CN2021/085308**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8-10**
because they relate to subject matter not required to be searched by this Authority, namely:

[1] Claims 8-10 relate to a treatment method, and belong to subject matter which does not require a search under PCT Rule 39 (iv). However, the applicant is expected to modify claims 8 and 9 as a "use of the compound according to any one of claims 1-5 or the pharmaceutical composition according to claim 6 or 7 in preparation of an immunosuppressant", modify claim 10 as a "use of the compound according to any one of claims 1 -5 or the pharmaceutical composition according to claim 6 or 7 in preparation of a medicament for preventing or treating immune-related diseases", and thus, the international search report is made on said basis.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/085308**

| Patent document cited in search report | | | Publication date (day/month/year) | Patent family member(s) | | | Publication date (day/month/year) |
|---|---|---|---|---|---|---|---|
| JP | 2012224564 | A | 15 November 2012 | None | | | |
| CN | 101123986 | A | 13 February 2008 | IL | 174595 | D0 | 20 August 2006 |
| | | | | US | 8592394 | B2 | 26 November 2013 |
| | | | | US | 2010093649 | A1 | 15 April 2010 |
| | | | | US | 8586564 | B2 | 19 November 2013 |
| | | | | JP | 2007533632 | A | 22 November 2007 |
| | | | | US | 2010221358 | A1 | 02 September 2010 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Nat. Prod.,* 2017, vol. 80 (6), 1734-1741
- Studies on the Purification. **XU JIE.** Analysis and Bioactivities of Cerebrosides from Sea Cucumbers. Ocean University, 2011
- **S. M. BERGE et al.** *J. Pharmaceutical Sciences,* 1977, vol. 66, 1-19
- Pro-drugs as Novel Delivery Systems. **T. HIGUCHI ; V. STELLA.** Bioreversible Carriers in Drug Design. American Pharmaceutical Association and Pergamon Press, 1987, vol. 14
- **J. RAUTIO et al.** Prodrugs: Design and Clinical Applications. *Nature Review Drug Discovery,* 2008, vol. 7, 255-270
- **S. J. HECKER et al.** Prodrugs of Phosphates and Phosphonates. *Journal of Medicinal Chemistry,* 2008, vol. 51, 2328-2345
- Remington: The Science and Practice of Pharmacy. Lippincott Williams & Wilkins, 2005
- Encyclopedia of Pharmaceutical Technology. Marcel Dekker, 1988